# EUROPEAN PATENT APPLICATION

(11) **EP 4 804 243 A2**
(43) Date of publication of application: **09.09.2026**
(21) Application number: 26156175.7
(22) Date of filing: 03.02.2026
(51) Int. Cl.: H01M 4/04, H01M 4/139, C12M 3/06

(54) **RECHARGEABLE BATTERY, MANUFACTURING METHOD THEREOF, AND FUNCTIONAL PARTICLE MANUFACTURING APPARATUS**

(30) Priority: 07.03.2025 KR 20250030086
(71) Applicant: SAMSUNG SDI CO., LTD., Yongin-si, Gyeonggi-do 17084 (KR)
(72) Inventor: LEE, Minho, Yongin-si, Gyeonggi-do 17084 (KR); KIM, Hyunwoo, Yongin-si, Gyeonggi-do 17084 (KR)
(74) Representative: Maiwald GmbH

(57) **Abstract**

A rechargeable battery including a positive substrate having a plurality of first protrusions, a negative substrate facing the positive substrate and having a plurality of second protrusions, and a plurality of functional particles between the plurality of first protrusions and the plurality of second protrusions, wherein each of the plurality of functional particles includes a first aggregate having a positive active material, a second aggregate having a negative active material, and a separating aggregate separating the first aggregate and the second aggregate and surrounding the first aggregate and the second aggregate, and the plurality of first protrusions penetrate the separating aggregate and contact the first aggregate and the plurality of second protrusions penetrate the separating aggregate and contact the second aggregate.

## Description

### BACKGROUND

### 1. Field

Embodiments relate to a rechargeable battery, and more particularly, to a rechargeable battery having a plurality of functional particles.

### 2. Description of the Related Art

Some rechargeable batteries are manufactured by manufacturing a positive electrode and a negative electrode through an active material slurry coating process, laminating or winding the positive electrode, the negative electrode and a separator to manufacture an electrode assembly, placing the electrode assembly inside a case, and injecting an electrolyte into the case and then sealing it.

### SUMMARY

An aspect is directed to a rechargeable battery including a positive substrate having a plurality of first protrusions, a negative substrate facing the positive substrate and having a plurality of second protrusions, and a plurality of functional particles between the plurality of first protrusions and the plurality of second protrusions, wherein each of the plurality of functional particles includes a first aggregate having a positive active material, a second aggregate having a negative active material, and a separating aggregate separating the first aggregate and the second aggregate and surrounding the first aggregate and the second aggregate, and the plurality of first protrusions penetrate the separating aggregate and contact the first aggregate and the plurality of second protrusions penetrate the separating aggregate and contact the second aggregate.

The positive substrate may include a first substrate having flexibility and the plurality of first protrusions may be on one side of the first substrate facing the negative substrate, and the negative substrate may include a second substrate having flexibility and the plurality of second protrusions may be on one side of the second substrate facing the positive substrate.

Each of the plurality of first protrusions and each of the plurality of second protrusions may have sharp ends and may have either a spike shape or a needle shape.

The first aggregate and the second aggregate may have a hemispherical shape, and the separating aggregate may include a barrier separating the first aggregate and the second aggregate and a protective layer surrounding the first aggregate and the second aggregate.

The plurality of functional particles may be arranged in a single layer and aligned such that the first aggregate faces the positive substrate and the second aggregate faces the negative substrate.

The second aggregate may further include a magnetic material.

The rechargeable battery according to some embodiments may further include an electrolyte surrounding the plurality of functional particles and a sealing part at an edge of the positive substrate and the negative substrate, wherein the separating aggregate may include a porous substrate holding the electrolyte in internal pores.

Another aspect is directed to a method of manufacturing a rechargeable battery, the method including manufacturing a plurality of functional particles, each including a first aggregate having a positive active material, a second aggregate having a negative active material, and a separating aggregate that separates the first aggregate and the second aggregate and surrounds the first aggregate and the second aggregate, manufacturing a positive substrate having a plurality of first protrusions and a negative substrate having a plurality of second protrusions, providing the plurality of functional particles on one substrate of the positive substrate and the negative substrate and aligning the plurality of functional particles to place the first aggregate and the second aggregate at designated positions, and arranging the other substrate of the positive substrate and the negative substrate on the plurality of functional particles.

Manufacturing the plurality of functional particles may include forming a positive slurry droplet including a positive active material and a negative slurry droplet including a negative active material in a flowing polymer solution, forming a polymer solution droplet surrounding the positive slurry droplet and negative slurry droplet in a flowing external fluid, and forming a first aggregate, a second aggregate, and a separating aggregate by solidifying and drying the positive slurry droplet, the negative slurry droplet, and the polymer solution droplet.

A positive slurry including the positive active material and a polar solvent may be intermittently discharged into the flowing polymer solution, the positive slurry may be polar and the flowing polymer solution may be non-polar, and the polar positive slurry may form the positive slurry droplet without mixing with the non-polar polymer solution.

A negative slurry including the negative active material and a polar solvent may be intermittently discharged into the flowing polymer solution, the negative slurry may be polar and the flowing polymer solution may be non-polar, and the polar negative slurry may form the negative slurry droplet without mixing with the non-polar polymer solution.

The external fluid may include a coagulant solution, the flowing polymer solution surrounding the positive slurry droplet and the negative slurry droplet may be discharged into the flowing external fluid, and the discharged polymer solution may be solidified to form the polymer solution droplet.

The positive substrate may be manufactured by a process of making a plurality of first protrusions by surface-treating one side of a first substrate having flexibility, and the negative substrate may be manufactured by a process of making a plurality of second protrusions by surface-treating one side of a second substrate having flexibility.

The one substrate of the positive substrate and the negative substrate may be the negative substrate including the plurality of second protrusions oriented upward, the plurality of functional particles may be provided in multiple layers over the plurality of second protrusions, and a scraper may push the plurality of functional particles to align the plurality of functional particles in a single layer.

The second aggregate may include a magnetic material, a magnet may be under the negative substrate, and the second aggregate may be oriented toward the negative substrate by magnetic force when the plurality of functional particles aligned in a single layer move over the magnet.

A vibration element may be proximate the magnet to vibrate the negative substrate, the plurality of functional particles may be bounced and then dropped due to vibration, and the second aggregates may be directed toward the negative substrate by magnetic force.

The other substrate of the positive substrate and the negative substrate may be the positive substrate including the plurality of first protrusions facing downward, the positive substrate and the negative substrate may be pressed toward each other while passing through a pair of calender rolls, and the plurality of first protrusions may contact the first aggregate and the plurality of second protrusions may contact the second aggregate due to mechanical force.

Embodiments are directed to a functional particle manufacturing apparatus including a first pipe unit having a first branch pipe and a second branch pipe and one discharge pipe, the first branch pipe and the second branch pipe being connected to the discharge pipe, a second pipe unit surrounding the discharge pipe and having a length greater than the discharge pipe, a polymer solution supply unit configured to supply polymer solution to the first branch pipe and the second branch pipe, a positive slurry supply unit configured to intermittently discharge positive slurry into the polymer solution of the first branch pipe, a negative slurry supply unit configured to intermittently discharge negative slurry into the polymer solution of the second branch pipe, and an external fluid supply unit configured to supply an external fluid containing a coagulant to the second pipe unit.

The first branch pipe and the second branch pipe may each have a substantially constant diameter, and may be positioned to be inclined at substantially the same angle with respect to the discharge pipe, and the discharge pipe may have a diameter that gradually decreases as a distance from the first branch pipe and the second branch pipe increases.

The second pipe unit may be configured to discharge a coagulated polymer solution droplet which may surround a positive slurry droplet and a negative slurry droplet, and may further include a dryer for drying the coagulated polymer solution droplet.

### BRIEF DESCRIPTION OF THE DRAWINGS

Features will become apparent to those of skill in the art by describing in detail exemplary embodiments with reference to the attached drawings in which:
FIG. 1 is a perspective view of a rechargeable battery according to an embodiment.
FIG. 2 is a cross-sectional view of the rechargeable battery shown in FIG. 1.
FIG. 3 is an exploded perspective view of the functional particles in the rechargeable battery illustrated in FIG. 1.
FIG. 4 is a process flowchart illustrating a method of manufacturing a rechargeable battery according to an embodiment.
FIG. 5 is a schematic diagram for explaining S10 of FIG. 4.
FIG. 6 is a schematic diagram for explaining S20 of FIG. 4.
FIG. 7 is a schematic diagram for explaining S30 and S40 of FIG. 4.

### DETAILED DESCRIPTION OF THE EMBODIMENTS

Example embodiments will now be described more fully hereinafter with reference to the accompanying drawings; however, they may be embodied in different forms and should not be construed as limited to the embodiments set forth herein. Rather, these embodiments are provided so that this disclosure will be thorough and complete, and will fully convey exemplary implementations to those skilled in the art.

In the drawing figures, the dimensions of layers and regions may be exaggerated for clarity of illustration. It will also be understood that when a layer or element is referred to as being "on" another layer or substrate, it can be directly on the other layer or substrate, or intervening layers may also be present. Further, it will be understood that when a layer is referred to as being "under" another layer, it can be directly under, and one or more intervening layers may also be present. In addition, it will also be understood that when a layer is referred to as being "between" two layers, it can be the only layer between the two layers, or one or more intervening layers may also be present. Like reference numerals refer to like elements throughout.

When the terms "about," "approximately" or "substantially" are used in this specification in connection with a numerical value, it is intended that the associated numerical value include a tolerance of ±10% around the stated numerical value. When ranges are specified, the range includes all values therebetween such as increments of 0.1%. As used herein, the term "or" is not an exclusive term, e.g., "A or B" would include A, B, or A and B.

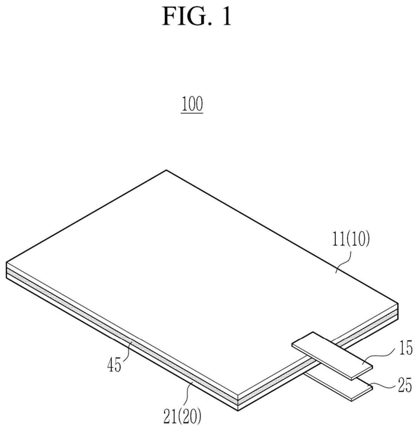

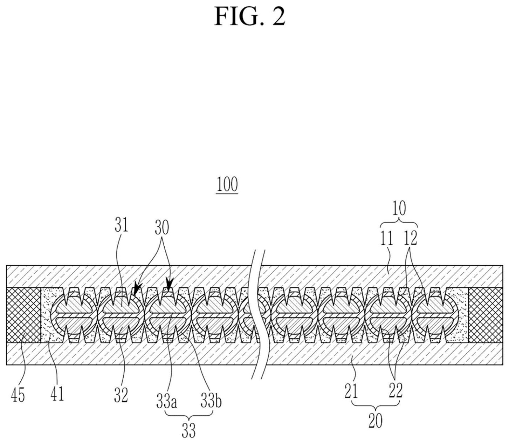

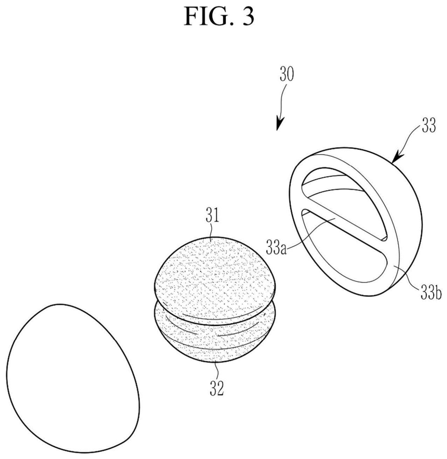

FIG. 1 is a perspective view of a rechargeable battery according to an embodiment. FIG. 2 is a cross-sectional view of the rechargeable battery shown in FIG. 1. FIG. 3 is an exploded perspective view of the functional particles in the rechargeable battery illustrated in FIG. 1.

Referring to FIGS. 1 to 3, a rechargeable battery 100 according to the present embodiment may include, e.g., a positive substrate 10 having a plurality of first protrusions 12 on one surface thereof, a negative substrate 20 having a plurality of second protrusions 22 on one surface thereof, and a plurality of functional particles 30 between the plurality of first protrusions 12 and the plurality of second protrusions 22. The rechargeable battery 100 may further include, e.g., an electrolyte 41 surrounding a plurality of functional particles 30 and a sealing part 45 at an edge of the positive substrate 10 and the negative substrate 20.

The positive substrate 10 and the negative substrate 20 may have high electrical conductivity. The positive substrate 10 may include, e.g., a first substrate 11 and a plurality of first protrusions 12 on one surface of the first substrate 11 facing the negative substrate 20. The negative substrate 20 may include, e.g., a second substrate 21 and a plurality of second protrusions 22 on one surface of the second substrate 21 facing the positive substrate 10.

The first substrate 11 and the second substrate 21 may have a constant thickness, or a substantially constant thickness, and may have flexibility that allows them to bend by an external force. In an implementation, the first substrate 11 and the second substrate 21 may be, e.g., composed of metal foil. The plurality of first protrusions 12 and the plurality of second protrusions 22 may have sharp ends, and may be, e.g., in the shape of spikes or needles. For example, as may be seen in FIG. 2, the sharp ends of the plurality of first protrusions 12 of the positive substrate 10 may face the negative substrate 20 and the sharp ends of the plurality of second protrusions 22 of the negative substrate 20 may face the positive substrate 10.

Each of the plurality of functional particles 30 may include, e.g., a first aggregate 31 including a positive active material, a second aggregate 32 including a negative active material, and a separating aggregate 33 separating the first aggregate 31 and the second aggregate 32 and surrounding the first aggregate 31 and the second aggregate 32.

The first aggregate 31 may be a drying body, e.g., a dried product, of a positive slurry droplet described below. The first aggregate 31 may include, e.g., a positive active material and may further include a binder or a conductive material. The second aggregate 32 may be a drying body of a negative slurry droplet described below. The second aggregate 32 may include a negative active material and may further contain a binder or a conductive material.

The positive active material may include, e.g., a lithium transition metal composite oxide. The lithium transition metal composite oxide may include, e.g., a lithium-nickel oxide, a lithium-cobalt oxide, a lithium-manganese oxide, a lithium-iron phosphate compound, or a cobalt-free lithium nickel-manganese oxide.

The negative active material may include, e.g., a carbon active material and a silicon active material. The carbon active material may include, e.g., natural graphite or artificial graphite. The silicon active material may include, e.g., a silicon-carbon composite active material, silicon oxide (SiOx, 0<x≤2), or silicon carbide (SiC).

In each of the first aggregate 31 and the second aggregate 32, the binder may include, e.g., an aqueous binder, a non-aqueous binder, or a dry binder, and the conductive material may include, e.g., a carbon material such as natural graphite, artificial graphite, carbon black, carbon fibers, carbon nanofibers, and carbon nanotubes; a metal material in the form of metal powder or metal fiber including copper, nickel, aluminum, and silver; and a conductive polymer such as a polyphenylene derivative.

The second aggregate 32 may further include a magnetic material. The magnetic material may be, e.g., iron oxide. In the manufacturing method of a rechargeable battery 100, described below, the magnetic material may function to align the plurality of functional particles in specific positions.

The separating aggregate 33 may be a drying body, e.g., a dried product, of a polymer solution droplet, which will be described below. The separating aggregate 33 may include a cell barrier 33a between the first aggregate 31 and the second aggregate 32, and a protective layer 33b surrounding the first aggregate 31 and the second aggregate 32. The cell barrier 33a may physically separate the first aggregate 31 and the second aggregate 32. The protective layer 33b may surround the first aggregate 31 and the second aggregate 32 and may have a constant thickness, or a substantially constant thickness, so that the first aggregate 31 and the second aggregate 32 are not exposed to the surface of the functional particles 30. For example, the first aggregate 31 and the second aggregate 32 are completely encased in the separating aggregate 33.

The separating aggregate 33 may be, e.g., made of a porous substrate. The separating aggregate 33 may include, e.g., polyethylene, polypropylene, polyethylene terephthalate, polypropylene terephthalate, polybutylene terephthalate, polyester, polycarbonate, or polyimide.

Each of the first aggregate 31 and the second aggregate 32 may be approximately hemispherical in shape, and the separating aggregate 33 may be approximately spherical in shape. The first aggregate 31, the second aggregate 32, and the separating aggregate 33 may have a size of, e.g., approximately tens of micrometers (µm) to several millimeters (mm) depending on process conditions.

The first aggregate 31 and the second aggregate 32 may have the same size (or substantially the same size) or different sizes. Although the first aggregate 31 and the second aggregate 32 have the same size in FIG. 3, the first aggregate 31 may be configured to be larger than the second aggregate 32 according to the characteristics of the rechargeable battery 100, and conversely, the second aggregate 32 may be configured to be larger than the first aggregate 31.

The plurality of functional particles 30 may be arranged in a single layer between the positive substrate 10 and the negative substrate 20. In an implementation, the plurality of functional particles 30 may be aligned in a specific state (e.g., position) such that the first aggregate 31 faces the positive substrate 10 and the second aggregate 32 faces the negative substrate 20.

One or more of the plurality of first protrusions 12 may pass through the separating aggregate 33 and contact the first aggregate 31 to be electrically connected to the first aggregate 31. The positive substrate 10 and a plurality of first aggregates 31 may constitute a positive electrode of the rechargeable battery 100

One or more of the plurality of second protrusions 22 may pass through the separating aggregate 33 and contact the second aggregate 32 to be electrically connected to the second aggregate 32. The negative substrate 20 and a plurality of second aggregates 32 may constitute the negative electrode of the rechargeable battery 100.

The electrolyte 41 may be, e.g., in a liquid, solid, or gel state and may surround the plurality of functional particles 30 between the positive substrate 10 and the negative substrate 20. The electrolyte 41 may include, e.g., a lithium salt, an organic solvent, or an additive. Due to the porous structure, the separating aggregate 33 may hold the electrolyte in the internal pores and may allow the lithium ions to pass therethrough.

The sealing part 45 may be at an edge of the positive substrate 10 and the negative substrate 20. The sealing part 45 may integrally bond the positive substrate 10 and the negative substrate 20 together and may prevent leakage of the electrolyte 41 by confining the electrolyte 41 in the inner space between the positive substrate 10 and the negative substrate 20.

An insulating layer may be on the outer surface of the positive substrate 10 (e.g., the upper surface with respect to the drawing) and the outer surface of the negative substrate 20 (e.g., the lower surface with respect to the drawing). The rechargeable battery 100 may include a positive electrode tab 15 and a negative electrode tab 25. The positive electrode tab 15 may be attached to the outer surface of the positive substrate 10. The negative electrode tab 25 may be attached to the outer surface of the negative substrate 20. The positive electrode tab 15 and the negative electrode tab 25 may be electrically connected to an object using the rechargeable battery 100 as a power source.

During the charging process of the rechargeable battery 100, lithium ions may be deintercalated from the first aggregate 31 and intercalated into the second aggregate 32. During the discharging process of the rechargeable battery 100, lithium ions may be deintercalated from the second aggregate 32 and intercalated into the first aggregate 31. Since the plurality of functional particles 30 may be surrounded by the positive substrate 10, the negative substrate 20, and the electrolyte 41, a stable charging/discharging function may be performed.

In FIGS. 1 and 2, the minimum unit of the rechargeable battery 100 is illustrated. Multiple rechargeable batteries may be connected in series or parallel to form a battery module. According to an embodiment, the rechargeable battery 100 is thin, light, and flexible enough to be easily bent, and thus can be usefully used as a power source for various devices such as wearable mobile devices.

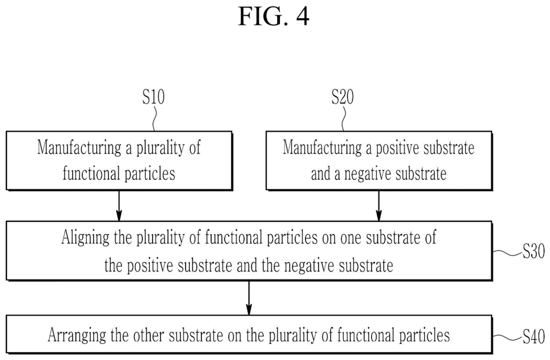

FIG. 4 is a process flowchart illustrating a method of manufacturing a rechargeable battery according to an embodiment.

Referring to FIG. 4, a method for manufacturing a rechargeable battery according to the present embodiment may include manufacturing a plurality of functional particles S10, manufacturing a positive substrate and a negative substrate S20, aligning a plurality of functional particles on one of the positive substrate and the negative substrate S30, and arranging the other one of the positive substrate and the negative substrate on the plurality of functional particles S40.

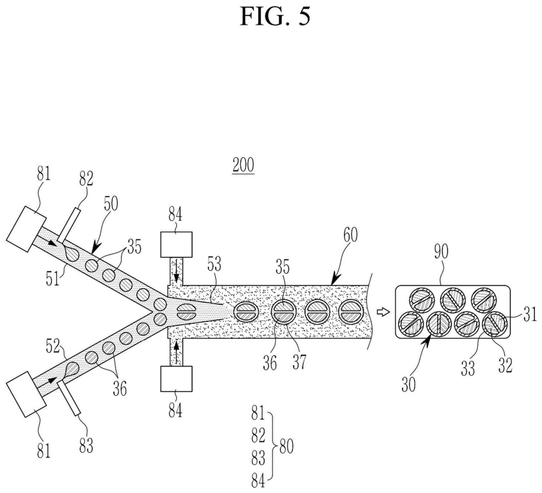

FIG. 5 is a schematic diagram for explaining S10 of FIG. 4.

Referring to FIG. 5, a functional particle manufacturing apparatus 200 according to one embodiment may be used in S10. A plurality of functional particles 30 may be manufactured using a microfluidic fabrication technique. The functional particle manufacturing apparatus 200 may include, e.g., a first pipe unit 50 including two branch pipes 51 and 52 and one discharge pipe 53, a second pipe unit 60 surrounding the discharge pipe 53, and a plurality of solution supply units 80 coupled to the first pipe unit 50 and the second pipe unit 60.

The first branch pipe 51 and the second branch pipe 52 may each have a constant diameter, or a substantially constant diameter, and may be inclined at the same angle, or substantially the same angle, with respect to the discharge pipe 53. The first branch pipe 51 and the second branch pipe 52 may have the same diameter, or substantially the same diameter, and the same length, or substantially the same length. The discharge pipe 53 may have a diameter that gradually decreases in the direction away from the first branch pipe 51 and the second branch pipe 52.

The second pipe unit 60 may be a straight pipe and may surround the discharge pipe 53 by a predetermined distance from the outer wall of the discharge pipe 53. The length of the second pipe unit 60 may be greater than the length of the discharge pipe 53. The discharge pipe 53 may contact one end (e.g., left end based on the drawing) of the second pipe unit 60 and may be located inside one side (e.g., left side based on the drawing) of the second pipe unit 60.

A plurality of solution supply units 80 may include a polymer solution supply unit 81 coupled to each of the first branch pipe 51 and the second branch pipe 52, a positive slurry supply unit 82 coupled to the first branch pipe 51, a negative slurry supply unit 83 coupled to the second branch pipe 52, and an external fluid supply unit 84 coupled to the second pipe unit 60. The plurality of solution supply units 80 may be configured as suitable pumping devices.

The polymer solution supply unit 81 may be connected to the end of the first branch pipe 51 and the end of the second branch pipe 52 and may supply the polymer solution into the interior of the first branch pipe 51 and the second branch pipe 52. The supplied polymer solution may flow at constant speed, or a substantially constant speed, toward the discharge pipe 53 inside the first branch pipe 51 and the second branch pipe 52. For example, as may be seen in FIG. 5, the first branch pipe 51 and the second branch pipe 52 may each be connected to the discharge pipe 53.

A polymer solution may include a polymer material and a solvent. Since the polymer material is the same as the material described in the separating aggregate description, a redundant description thereof will be omitted. The solvent may be, e.g., an organic solvent including dichloromethane.

In the first branch pipe 51, the positive slurry supply unit 82 may be located at a distance from the polymer solution supply unit 81 along the flow direction of the polymer solution and may intermittently emit the positive slurry with the flowing polymer solution.

The positive slurry may include a positive active material and a solvent and may further include a binder or a conductive material. Since the positive active material, the binder, and the conductive material may be the same as those described in the first aggregate description, redundant descriptions will be omitted. The solvent may be, e.g., an organic solvent including N-methyl-2-pyrrolidone NMP or the like, or an aqueous solvent including water.

The polymer solution and the positive slurry may have an immiscible fluid property that is not spontaneously mixed. The positive slurry may be agglomerated into a droplet without being mixed with a polymer solution, and the agglomerated positive slurry droplets 35 may move along with the polymer solution. The size of the positive slurry droplets 35 may be adjusted according to the flow rate of the positive slurry emitted by the positive slurry supply unit 82.

In the second branch pipe 52, the negative slurry supply unit 83 may be located at a distance from the polymer solution supply unit 81 along the flow direction of the polymer solution and may intermittently emit the negative slurry into the flowing polymer solution.

The negative slurry may include a negative active material, a magnetic material, and a solvent, and may further include a binder or a conductive material. Since the negative active material, the magnetic material, the binder, and the conductive material may be the same as those described in the second aggregate description, redundant descriptions will be omitted. The solvent may be, e.g., an aqueous solvent including water.

The polymer solution and the negative slurry may have an immiscible fluid characteristic that is not spontaneously mixed. The negative slurry may be agglomerated into droplets without being mixed with a polymer solution, and the agglomerated negative slurry droplets 36 may move along with the polymer solution. The size of the negative slurry droplets 36 may be adjusted according to the flow rate of the negative slurry emitted by the negative slurry supply unit 83.

The solvent of the positive slurry and the solvent of the negative slurry may be polar solvents, and the positive slurry and the negative slurry may have polarities. In an implementation, the polymer solution may be non-polar. Therefore, each of the positive slurry droplets 35 and the negative slurry droplets 36 may not be spontaneously mixed with the polymer solution, and the positive slurry droplets 35 and the negative slurry droplets 36 may not be mixed with each other because physical contact between them may be blocked by the polymer solution.

The positive slurry droplets 35 and the negative slurry droplets 36 may become close to each other in the discharge pipe 53 and may be slightly flattened without maintaining a ball shape in the discharge pipe 53.

The external fluid supply unit 84 may be coupled to one end (e.g., left end based on the drawing) of the second pipe unit 60 surrounding the discharge pipe 53 and may supply an external fluid to the inside of the second pipe unit 60. The supplied external fluid may flow at constant speed, or a substantially constant speed, along the second pipe unit 60.

The external fluid may include, e.g., a coagulant solution for coagulating the polymer solution. The coagulant solution may function to coagulate a polymer solution using a chemical reaction or dehydration action. The external fluid may include, e.g., a coagulant and a solvent, and various kinds of coagulants and solvents may be used according to components of the polymer solution.

The discharge pipe 53 may discharge a polymer solution surrounding the negative slurry droplets 36 and the positive slurry droplets 35, and the polymer solution may be aggregated into droplets by the flow of an external fluid. When the positive slurry droplets 35 and the negative slurry droplets 36 are referred to as primary droplets, a secondary droplet of a polymer solution surrounding two primary droplets may be formed by the flow of an external fluid.

The positive slurry droplets 35, the negative slurry droplets 36, and the polymer solution droplets 37 may be gradually solidified while flowing along an external fluid. The size of the polymer solution droplets 37 and the thickness of the polymer solution droplets 37 may be adjusted according to the flow rate of the external fluid. In this case, the thickness of the polymer solution droplets 37 means the thickness of a portion to be a protective layer 33b (see FIG. 3) later.

A configuration of separating and collecting solidified polymer solution droplets from an external fluid may be provided at the opposite end (e.g., right side based on the drawing) of the second pipe unit 60. In an implementation, the functional particle manufacturing apparatus 200 may further include, e.g., a dryer 90 for drying the coagulated polymer solution droplets. The coagulated polymer solution droplets may be dried by a dryer 90 to complete a plurality of functional particles 30 including, e.g., a first aggregate 31, a second aggregate 32, and a separating aggregate 33.

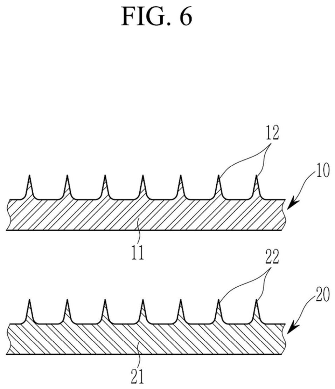

FIG. 6 is a schematic diagram for explaining S20 of FIG. 4.

Referring to FIG. 6, the positive substrate 10 in S20 may be manufactured by a process of preparing a first substrate 11, and processing, e.g., making, a plurality of first protrusions 12 by surface-treating one surface of the first substrate 11. The negative substrate 20 may be manufactured by a process of preparing a second substrate 21 and processing, e.g., making, a plurality of second protrusions 22 by surface-treating one surface of the second substrate 21.

The first substrate 11 and the second substrate 21 may be flexible and may be, e.g., composed of metal foil. The surface treatment of the first substrate 11 and the second substrate 21 may be performed by, e.g., a chemical surface treatment using an etchant, a physical surface treatment using a mold, or a surface treatment using a laser. A plurality of first protrusions 12 and a plurality of second protrusions 22 may have, e.g., a spike or a needle shape.

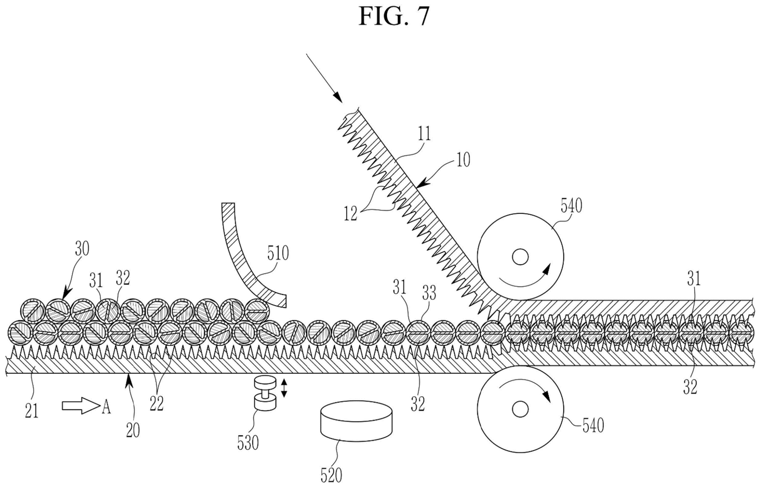

FIG. 7 is a schematic diagram for explaining S30 and S40 of FIG. 4.

Referring to FIG. 7, in S30, a plurality of functional particles 30 may be supplied the positive substrate 10 or the negative substrate 20. In an implementation, a plurality of functional particles 30 may be supplied over, e.g., on top of, a plurality of second protrusions 22 on the negative substrate 20. Immediately after being supplied, the plurality of functional particles 30 may be stacked in two or more layers and may exist in a randomly oriented state where the positions of the first aggregate 31 and the second aggregate 32 are not constant.

The negative substrate 20 may be moved at a constant speed, or substantially constant speed, by a transport device. In FIG. 7, the moving direction of the negative substrate 20 is indicated by an arrow A. The plurality of functional particles 30 may be aligned in a single layer by the scraper 510.

The scraper 510 may be on the upper side of the negative substrate 20 and may be formed of a polymer film structure having a predetermined elasticity. The position of the scraper 510 may be set to face the functional particles 30 of two or more layers excluding the first layer among the multi-layered functional particles 30. Among the plurality of functional particles 30 supplied to the negative substrate 20, functional particles 30 of two or more layers may be pushed out by the scraper 510, and only the functional particles 30 of the first layer may pass through the scraper 510 and be aligned as a single layer on the negative substrate 20.

The plurality of functional particles 30 may be aligned (e.g., oriented) in a predetermined direction by a magnet 520 and a vibration element 530 may be additionally used in this process. The vibration element 530 and the magnet 520 may be located under the negative substrate 20. The vibration element 530 and the magnet 520 may be sequentially along the moving direction of the negative substrate 20. The vibration element 530 may vibrate the negative substrate 20 by periodically applying an impact to the negative substrate 20. The magnet 520 may apply a magnetic force to a plurality of functional particles 30.

The plurality of functional particles 30 aligned in a single layer on the negative substrate 20 may bounce slightly due to the vibration, and the second aggregate 32 including magnetic material may be directed downward by attractive force, e.g., magnetic force, in the process of falling down. A plurality of functional particles 30 may be aligned in a single layer on the negative substrate 20 and may be constantly, or substantially constantly, oriented so that the second aggregate 32 faces the negative substrate 20.

In S40, the positive substrate 10 may be supplied over a plurality of functional particles 30. The positive substrate 10 may be positioned so that the plurality of first protrusions 12 face a plurality of functional particles 30. The positive substrate 10 and the negative substrate 20 may face each other with a plurality of functional particles 30 therebetween.

The positive substrate 10 and the negative substrate 20 may be pressed along the thickness direction so that the distance between them decreases by roll pressing. In an implementation, the positive substrate 10 and the negative substrate 20 may be pressed toward each other while passing between the pair of calender rolls 540. In this process, one or more of the plura\lity of first protrusions 12 may penetrate the separating aggregate 33 and contact the first aggregate 31, and one or more of the plurality of second protrusions 22 may penetrate the separating aggregate 33 and contact the second aggregate 32.

In FIG. 7, the distance between the magnet 520 and the calender roll 540 is enlarged for convenience of illustration, but the distance between the magnet 520 and the calender roll 540 may be very short. Since the plurality of functional particles 30 may be oriented such that the second aggregate 32 faces downward and the first aggregate 31 faces upward by the magnet 520, the plurality of first protrusions 12 and the first aggregate 31 may be easily electrically connected and the plurality of second protrusions 22 and the second aggregate 32 may be easily electrically connected by a simple pressing operation of the positive substrate 10 and the negative substrate 20.

Referring again to FIG. 2, an electrolyte 41 may be between a plurality of functional particles 30 after S40, and a sealing part 45 may be at the edge of the positive substrate 10 and the negative substrate 20.

In an implementation, when the electrolyte 41 is in a liquid state, a sealing material may be applied along the edge of the positive substrate 10 and the negative substrate 20 except for a electrolyte injection port to form a sealing part 45. Subsequently, a liquid electrolyte 41 may be injected between the positive substrate 10 and the negative substrate 20 through the electrolyte injection port, and after the electrolyte 41 is injected, the electrolyte injection port may be sealed with a sealing material.

By way of summation and review, rechargeable batteries may be manufactured through a multi-step detailed process, which may increase manufacturing complexity and may take a lot of time to manufacture. In addition, it may be difficult for a typical rechargeable battery to be flexible due to a rigid case.

The present disclosure is to provide a rechargeable battery capable of simplifying the manufacturing process and capable of being flexibly deformable, and a method for manufacturing such a rechargeable battery.

According to an embodiment, the rechargeable battery may be thin, light, and flexible enough to be easily bent, and thus may be used as a power source for various devices such as wearable mobile devices. In an implementation, functional particles may be easily prepared using a microfluidic manufacturing technique, and functional particles may be easily aligned using magnetic force.

Example embodiments have been disclosed herein, and although specific terms are employed, they are used and are to be interpreted in a generic and descriptive sense only and not for purpose of limitation. In some instances, as would be apparent to one of ordinary skill in the art as of the filing of the present application, features, characteristics, and/or elements described in connection with a particular embodiment may be used singly or in combination with features, characteristics, and/or elements described in connection with other embodiments unless otherwise specifically indicated. Accordingly, it will be understood by those of skill in the art that various changes in form and details may be made without departing from the scope of the present invention as set forth in the following claims.

## Claims

1. A rechargeable battery (100), comprising:
a positive substrate (10) having a plurality of first protrusions (12);
a negative substrate (20) facing the positive substrate (10) and having a plurality of second protrusions (22); and
a plurality of functional particles (30) between the plurality of first protrusions (12) and the plurality of second protrusions (22),
wherein:
each of the plurality of functional particles (30) includes a first aggregate (31) having a positive active material, a second aggregate (32) having a negative active material, and a separating aggregate (33) separating the first aggregate (31) and the second aggregate (32) and surrounding the first aggregate (31) and the second aggregate (32), and
the plurality of first protrusions (12) penetrate the separating aggregate (33) and contact the first aggregate (31) and the plurality of second protrusions (22) penetrate the separating aggregate (33) and contact the second aggregate (32).

2. The rechargeable battery (100) as claimed in claim 1, wherein:
the positive substrate (10) includes a first substrate (11) having flexibility and the plurality of first protrusions (12) are on one side of the first substrate (11) facing the negative substrate (20), and
the negative substrate (20) includes a second substrate (21) having flexibility and the plurality of second protrusions (22) are on one side of the second substrate (21) facing the positive substrate (10), wherein preferably each of the plurality of first protrusions (12) and each of the plurality of second protrusions (22) have sharp ends and have either a spike shape or a needle shape.

3. The rechargeable battery (100) as claimed in claim 1 or 2, wherein:
the first aggregate (31) and the second aggregate (32) have a hemispherical shape, and
the separating aggregate (33) includes a barrier (33a) separating the first aggregate (31) and the second aggregate (32) and a protective layer (33b) surrounding the first aggregate (31) and the second aggregate (32).

4. The rechargeable battery (100) as claimed in any one of the preceding claims, wherein the plurality of functional particles (30) are arranged in a single layer and aligned such that the first aggregate (31) faces the positive substrate (10) and the second aggregate (32) faces the negative substrate (20), wherein preferably the second aggregate (32) further includes a magnetic material.

5. The rechargeable battery (100) as claimed in any one of the preceding claims, further comprising:
an electrolyte (41) surrounding the plurality of functional particles (30); and
a sealing part (45) at an edge of the positive substrate (10) and the negative substrate (20),
wherein the separating aggregate (33) includes a porous substrate holding the electrolyte (41) in internal pores.

6. A method of manufacturing a rechargeable battery (100), the method comprising:
manufacturing (S10) a plurality of functional particles (30), each including a first aggregate (31) having a positive active material, a second aggregate (32) having a negative active material, and a separating aggregate (33) that separates the first aggregate (31) and the second aggregate (32) and surrounds the first aggregate (31) and the second aggregate (32);
manufacturing (S20) a positive substrate (10) having a plurality of first protrusions (12) and a negative substrate (20) having a plurality of second protrusions (22);
providing the plurality of functional particles (30) on one substrate of the positive substrate (10) and the negative substrate (20) and aligning (S30) the plurality of functional particles (30) to place the first aggregate (31) and the second aggregate (32) at designated positions; and
arranging (S40) the other substrate of the positive substrate (10) and the negative substrate (20) on the plurality of functional particles (30).

7. The method of manufacturing a rechargeable battery (100) as claimed in claim 6, wherein manufacturing the plurality of functional particles (30) includes:
forming a positive slurry droplet (35) including a positive active material and a negative slurry droplet (36) including a negative active material in a flowing polymer solution,
forming a polymer solution droplet (37) surrounding the positive slurry droplet (35) and negative slurry droplet (36) in a flowing external fluid, and
forming a first aggregate (31), a second aggregate (32), and a separating aggregate (33) by solidifying and drying the positive slurry droplet (35), the negative slurry droplet (36), and the polymer solution droplet (37).

8. The method of manufacturing a rechargeable battery (100) as claimed in claim 7, wherein:
a positive slurry including the positive active material and a polar solvent is intermittently discharged into the flowing polymer solution,
the positive slurry is polar and the flowing polymer solution is non-polar, and
the polar positive slurry forms the positive slurry droplet (35) without mixing with the non-polar polymer solution; and/or
a negative slurry including the negative active material and a polar solvent is intermittently discharged into the flowing polymer solution,
the negative slurry is polar and the flowing polymer solution is non-polar, and
the polar negative slurry forms the negative slurry droplet (36) without mixing with the non-polar polymer solution.

9. The method of manufacturing a rechargeable battery (100) as claimed in claim 7 or 8, wherein:
the external fluid includes a coagulant solution,
the flowing polymer solution surrounding the positive slurry droplet (35) and the negative slurry droplet (36) is discharged into the flowing external fluid, and
the discharged polymer solution is solidified to form the polymer solution droplet (37).

10. The method of manufacturing a rechargeable battery (100) as claimed in any one of the claims 8 or 9, wherein:
the positive substrate (10) is manufactured by a process of making a plurality of first protrusions (12) by surface-treating one side of a first substrate (11) having flexibility, and
the negative substrate (20) is manufactured by a process of making a plurality of second protrusions (22) by surface-treating one side of a second substrate (21) having flexibility.

11. The method of manufacturing a rechargeable battery (100) as claimed in any one of the claims 8 to 10, wherein:
the one substrate of the positive substrate (10) and the negative substrate (20) is the negative substrate (20) including the plurality of second protrusions (22) oriented upward,
the plurality of functional particles (30) are provided in multiple layers over the plurality of second protrusions (22), and
a scraper (510) pushes the plurality of functional particles (30) to align the plurality of functional particles (30) in a single layer.

12. The method of manufacturing a rechargeable battery (100) as claimed in claim 11, wherein:
the second aggregate (32) includes a magnetic material,
a magnet (520) is under the negative substrate (20), and
the second aggregate (32) is oriented toward the negative substrate (20) by magnetic force when the plurality of functional particles (30) aligned in the single layer move over the magnet (520).

13. The method of manufacturing a rechargeable battery (100) as claimed in claim 12, wherein:
a vibration element (530) is proximate the magnet (520) to vibrate the negative substrate (20),
the plurality of functional particles (30) are bounced and then dropped due to vibration, and
the second aggregates (32) are directed toward the negative substrate (20) by magnetic force.

14. The method of manufacturing a rechargeable battery (100) as claimed in claim 12 or 13, wherein:
the other substrate of the positive substrate (10) and the negative substrate (20) is the positive substrate (10) including the plurality of first protrusions (12) facing downward,
the positive substrate (10) and the negative substrate (20) are pressed toward each other while passing through a pair of calender rolls, and
the plurality of first protrusions (12) contact the first aggregate (31) and the plurality of second protrusions (22) contact the second aggregate (32) due to mechanical force.

15. A functional particle (30) manufacturing apparatus, comprising:
a first pipe unit (50) having a first branch pipe (51) and a second branch pipe (52) and one discharge pipe (53);
the first branch pipe (51) and the second branch pipe (52) being connected to the discharge pipe (53);
a second pipe unit (60) surrounding the discharge pipe (53) and having a length greater than the discharge pipe (53);
a polymer solution supply unit (81) configured to supply polymer solution to the first branch pipe (51) and the second branch pipe (52);
a positive slurry supply unit (82) configured to intermittently discharge positive slurry into the polymer solution of the first branch pipe (51);
a negative slurry supply unit (83) configured to intermittently discharge negative slurry into the polymer solution of the second branch pipe (52); and
an external fluid supply unit (84) configured to supply an external fluid containing a coagulant to the second pipe unit (60).
